# EUROPÄISCHE PATENTANMELDUNG

(11) **EP 1 543 766 A1**
(43) Veröffentlichungstag der Anmeldung: **22.06.2005**
(21) Anmeldenummer: 04027673.5
(22) Anmeldetag: 22.11.2004
(51) Int. Cl.: A61B 1/04

(54) **System und Verfahren zur In Vivo Positions-und Orientierungsbestimmung einer Endoskopie-Kapsel**

(30) Priorität: 19.12.2003 DE 10359981
(71) Anmelder: SIEMENS AKTIENGESELLSCHAFT, 80333 München (DE)
(72) Erfinder: Graumann, Rainer, Dr., 91315 Höchstadt (DE)

(57) **Zusammenfassung**

Die vorliegende Erfindung bezieht sich auf ein System zur In Vivo Positions- und Orientierungsbestimmung einer Endoskopie-Kapsel (1) im Rahmen einer kabellosen Endoskopie, aufweisend
- einen Magnetfeld-Generator (12) zur Erzeugung eines elektromagnetischen HF-3D-Gradientenfeldes im zu untersuchenden Bereich,
- eine Endoskopie-Kapsel (1) mit integrierter Empfangseinheit (20) zur Ermittlung des jeweils aktuellen positions- und orientierungsspezifischen HF-3D-Gradientenfeldwertes der Endoskopie-Kapsel (1),
- eine Navigations-Einheit (15) zum räumlichen Zuordnen des aktuellen HF-3D-Gradientenfeld-Wertes, und
- eine Visualisierungs-Einheit (8) zur Darstellung der in einer definierten Position und Orientierung aufgenommenen anatomischen Bilder auf einem Bildschirm.

## Beschreibung

Die vorliegende Erfindung bezieht sich allgemein auf die kabellose Endoskopie mit einer Endoskopie-Kapsel bzw. mit einem Endoroboter zur Durchführung minimalinvasiver Diagnosen und Eingriffe im Körperinneren, vorzugsweise im Gastrointestinaltrakt eines Patienten. Dabei bezieht sich die vorliegende Erfindung insbesondere auf ein System sowie ein Verfahren zur Bestimmung der In Vivo Position der Endoskopie-Kapsel bzw. des Endoroboters.

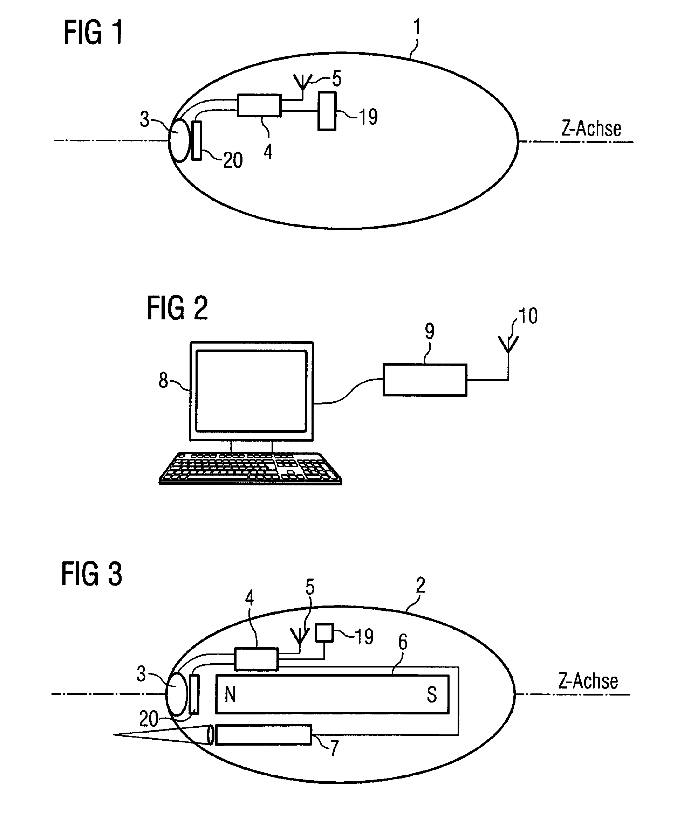

In der endoskopischen Diagnose wird neuerdings eine kabellose Endoskopiekapsel eingesetzt, wie sie in Figur 1 dargestellt ist. Sie verfügt über eine Kamera 3, eine interne Spannungsversorgung, einen Speicherbaustein 19 sowie eine Sendeeinheit 4 mit Antenne 5. Die Endoskopie-Kapsel 1 wird vom Patienten oral eingenommen und durchläuft dann den Verdauungstrakt auf natürlichem Wege. Über die interne Sendeeinheit 4 und die Antenne 5 ist die Endoskopie-Kapsel in der Lage, von der Kamera 3 aufgenommene Bilder kontinuierlich aus dem Verdauungstrakt nach außen zu senden (beispielsweise ein Bild pro Sekunde), die dann gemäß Figur 2 über die Antenne 10 von einer Empfangseinheit 9 aufgenommen und gespeichert und letztlich auf einer Visualisierungs-Einheit 8 dargestellt werden.

Aus US 6,240,312 B1 ist ein ebenfalls kabelloser Endoroboter 2 bekannt, der im Vergleich zur Endoskopie-Kapsel 1 zusätzlich einen ablationsfähigen Laser 7 aufweist und zudem von außen durch Variation externer Magnetfelder aktiv gesteuert werden kann. Die Steuerung eines solchen Endoroboters 2 im Körperinneren wird durch ein Magnetfeld-Steuerungssystem realisiert und ist in der Patentschrift DE 101 42 253 C1 ausführlich beschrieben.

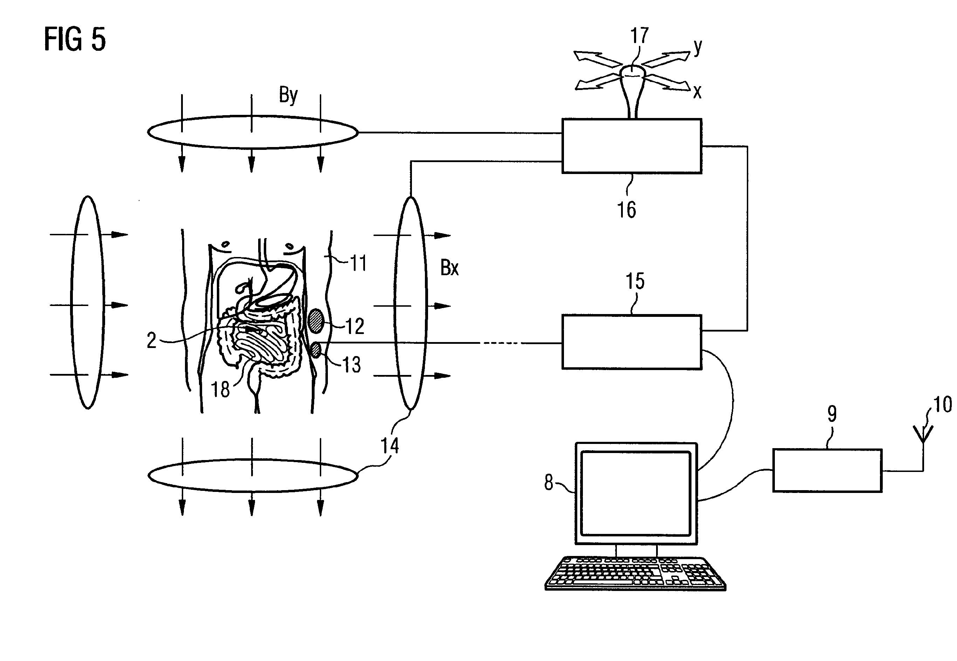

Zur aktiven Steuerung des Endoroboters 2, das heißt zur ferngesteuerten Bewegung und Orientierung im Körperinneren, ist der Endoroboter 2 gemäß Figur 3 mit einem Linearmagneten (Stabmagnet oder lineare Spule 6) versehen, der gemäß Figur 5 auf außen angelegte variierbare 3D-Gradientenfelder 14 entsprechend reagiert, in dem auf ihn eine lineare Kraft und ein Drehmoment ausgeübt wird. Navigiert wird der Endoroboter 2 durch den Anwender mittels eines Krafteingabegerätes 16, zum Beispiel einer sogenannten 6D-Maus, durch Neigen nach vorne/hinten und rechts/links sowie durch Drücken oder Heben bzw. durch Drehen eines Eingabehebels 17. Wiederum werden die von der Endoroboter-Kamera 3 aufgenommenen Bilder über die Sendeeinheit 4 und die Antenne 5 nach außen gesendet und nach Übertragung über die Antenne 10 und die Empfangseinheit 9 auf der Visualisierungs-Einheit 8 dargestellt.

Wie bereits bemerkt, sind Endoskopie-Kapsel 1 und Endoroboter 2 mit einer integrierten (Mini)Kamera ausgestattet, die auf dem (gesteuerten) Weg durch den Magen-Darm-Trakt möglicherweise über mehrere Stunden hinweg (6 bis 8 Stunden) kontinuierlich Bilder aus dem Körperinneren aussendet und damit beispielsweise die endoskopische Betrachtung des gesamten Dünndarms ermöglicht mit einer sehr hohen Auffindungsrate für krankhafte Veränderungen.

Das Problem, das sich dabei stellt, besteht in der korrekten Bildzuordnung zu anatomischen Strukturen, die aus einer bestimmten Position und Orientierung der Endoskopie-Kapsel 1 bzw. des Endoroboters 2 aufgenommen worden sind. Eine Bildzuordnung erfolgt derzeit ausschließlich auf Basis der vorhandenen anatomischen Kenntnisse des Anwenders.

Aufgabe der vorliegenden Erfindung ist es daher, ein System und ein Verfahren bereitzustellen, mit dem Bildaufnahmen der Endoskopie-Kapsel 1 bzw. des Endoroboters 2 der jeweiligen Position und Orientierung der Endoskopie-Kapsel 1 bzw. des Endoroboters 2 zum Zeitpunkt der Aufnahme direkt zugeordnet werden können.

Diese Aufgabe wird gemäß der Erfindung durch die Merkmale der unabhängigen Ansprüche gelöst. Die abhängigen Ansprüche bilden den zentralen Gedanken der Erfindung in besonders vorteilhafter Weise weiter.

Erfindungsgemäß wird daher ein System beansprucht zur In Vivo Positions- und Orientierungsbestimmung einer Endoskopie-Kapsel im Rahmen einer kabellosen Endoskopie aufweisend
- einen Magnetfeld-Generator zur Erzeugung eines elektromagnetischen HF-3D-Gradientenfeldes im zu untersuchenden Bereich,
- eine Endoskopie-Kapsel mit integrierter Empfangseinheit zur Ermittlung des jeweils aktuellen positions- und orientierungsspezifischen HF-3D-Gradientenfeldwertes der Endoskopie-Kapsel,
- eine Navigations-Einheit zum räumlichen Zuordnen des aktuellen HF-3D-Gradientenfeld-Wertes, und
- eine Visualisierungs-Einheit zur Darstellung der in einer definierten Endoskopie-Kapsel-Position und -Orientierung aufgenommenen anatomischen Bilder auf einem Bildschirm.

Dabei kann die Endoskopie-Kapsel erfindungsgemäß auch als Endoroboter ausgebildet sein.

In einer möglichen Ausführungsform der vorliegenden Erfindung weist die Endoskopie-Kapsel einen Speicherbaustein auf, welcher die zu der jeweiligen anatomischen Bildaufnahme korrespondierenden positions- und orientierungsspezifischen HF-3D-Gradientenfeldwerte abspeichert, die dann von der Navigations-Einheit gelesen werden.

In einer weiteren Ausführungsform weist die Endoskopie-Kapsel eine Sendeeinheit auf, mittels der die zu der jeweiligen anatomischen Bildaufnahme korrespondierenden positions- und orientierungsspezifischen HF-3D-Gradientenfeldwerte an die Navigations-Einheit gesendet werden.

Vorteilhaft stellt die integrierte Empfangseinheit eine Sensorspule dar.

Weiterhin vorteilhaft ist, wenn der Magnetfeld-Generator am Patienten befestigt ist und von diesem über einen längeren Zeitraum mobil mitgeführt wird.

In diesem Fall ist es auch sinnvoll, die Endoskopie-Kapsel-Empfangseinheit ebenfalls am Patienten zu befestigen und von diesem mitführen zu lassen.

Erfindungsgemäß ermöglicht das Navigationssystem eine Zuordnung der anatomischen Bilder zur jeweiligen Position und Orientierung der Endoskopie-Kapsel.

Erfindungsgemäß erfolgt die Darstellung der Endoskopie-Kapsel auf der Visualisierungs-Einheit.

In einer weiteren möglichen Ausgestaltung der Erfindung ermöglicht die Visualisierungs-Einheit eine parallele Darstellung von Endoskopie-Kapsel und der bei dieser Position und Orientierung gewonnenen anatomischen Aufnahme.

Ferner wird erfindungsgemäß ein Verfahren beansprucht zur In Vivo Positions- und Orientierungsbestimmung einer Endoskopie-Kapsel im Rahmen einer kabellosen Endoskopie, gekennzeichnet durch folgende Schritte:
- Erzeugen eines elektromagnetischen HF-3D-Gradientenfeldes im zu untersuchenden Bereich mittels einem Magnetfeld-Generator,
- Ermitteln des jeweils aktuellen positions- und orientierungsspezifischen HF-3D-Gradientenfeld-Wertes der Endoskopie-Kapsel mit einer integrierten Empfangseinheit,
- räumliches Zuordnen des aktuellen HF-3D-Gradientenfeld-Wertes mittels einer Navigations-Einheit, und
- Visualisieren der in einer definierten Position und Orientierung aufgenommenen anatomischen Bilder auf einem Bildschirm.

Dabei kann die Endoskopie-Kapsel erfindungsgemäß auch als Endoroboter ausgebildet sein.

Erfindungsgemäß werden die zu der jeweiligen anatomischen Bildaufnahme korrespondierenden positions- und orientierungsspezifischen HF-3D-Gradientenfeldwerte der Endoskopie-Kapsel in einem Speicherbaustein abgespeichert.

In einer weiteren erfindungsgemäßen Ausgestaltung der vorliegenden Erfindung werden die zu der jeweiligen anatomischen Bildaufnahme korrespondierenden positions- und orientierungsspezifischen HF-3D-Gradientenfeldwerte mittels einer in der Endoskopie-Kapsel integrierten Sendeeinheit an die Navigations-Einheit gesendet.

Vorteilhaft wird als integrierte Empfangseinheit eine Sensorspule verwendet.

Weiterhin vorteilhaft ist es, auf der Visualisierungs-Einheit auch die Darstellung der Endoskopie-Kapsel vorzunehmen.

In einer möglichen Ausgestaltung der Erfindung wird der Magnetfeld-Generator am Patienten befestigt und von diesem über einen längeren Zeitraum mobil mitgeführt.

Erfindungsgemäß erfolgt mittels der Navigationseinheit eine Zuordnung der anatomischen Bilder zur jeweiligen Endoskopie-Kapsel-Position und -Orientierung.

Weitere Vorteile, Merkmale und Eigenschaften der vorliegenden Erfindung werden nun anhand von Ausführungsbeispielen, bezugnehmend auf die begleitenden Zeichnungen, näher erläutert:
- Figur 1: zeigt eine Endoskopie-Kapsel mit integrierter Kamera, Speicherbaustein sowie Sendeeinheit und Antenne zur Bildübertragung.
- Figur 2: zeigt eine Visualisierungs-Einheit mit Antenne und Empfangseinheit zur Darstellung der Bilder.
- Figur 3: zeigt einen Endoroboter mit integrierter Kamera, Speicherbaustein, Sendeeinheit und Antenne zur Bildübertragung sowie mit einem ablationsfähigen Laser und Linearmagneten zur aktiven Navigation.
- Figur 4: zeigt eine Endoskopie-Kapsel im Dünndarm eines Patienten mit Magnetfeld-Generator, Empfangseinheit und Navigations-Einheit.
- Figur 5: zeigt einen Endoroboter im Dünndarm eines Patienten mit Magnetfeld-Steuerungssystem und Navigations-Einheit.

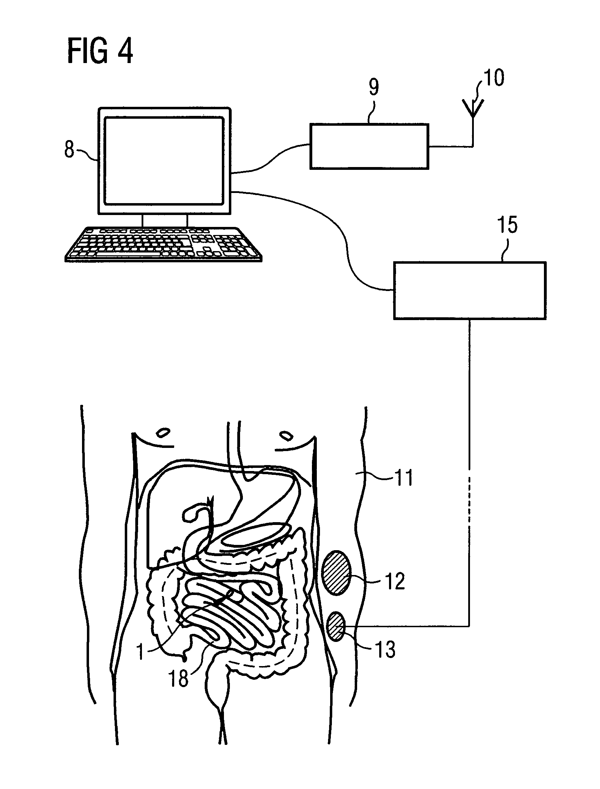

Die vorliegende Erfindung betrifft ein System bzw. ein Verfahren, welche die Zuordnung einer Bildaufnahme der Kamera 3 zur jeweiligen räumlichen Position und Orientierung der Endoskopie-Kapsel bzw. des Endoroboters während dieser Bildaufnahme ermöglichen. Das erfindungsgemäße System wird anhand der Figuren 4 und 5 erläutert, wobei in Figur 4 eine Endskopie-Kapsel verwendet wird und in Figur 5 die Endoskopie-Kapsel als navigierbarer Endoroboter ausgebildet ist.

Das erfindungsgemäße System umfasst einen Magnetfeldgenerator 12 welcher sich außerhalb des Körpers befindet und in dem Bereich in dem sich die Endoskopie-Kapsel bzw. der Endoroboter bewegt und Bilder aufnimmt ein stark variierendes magnetisches Wechselfeld erzeugt (beispielsweise ein Gradientenfeld oder ein Quadropolfeld).

Die Frequenz dieses magnetischen Wechselfeldes liegt in der Größenordnung von einigen kHz, so dass dieses Wechselfeld den menschlichen Körper nahezu störungsfrei durchdringt. Um die Endoskopie-Kapsel bzw. den Endoroboter dennoch für dieses Wechselfeld sensibel zu machen, ist die Endoskopie-Kapsel bzw. der Endoroboter mit einer Sensorspule 20 ausgestattet, welche so dimensioniert ist, dass das magnetische Wechselfeld von dieser Sensorspule 20 erfasst wird. Die Sensorspule 20 besitzt 5 oder 6 Freiheitsgrade, die in Abhängigkeit des räumlich stark variierenden Wechselfeldes gemessen werden. Die Messung definiert die Position und Orientierung der Sensorspule 20 im Wechselfeld und damit die Position und Orientierung der Endoskopie-Kapsel bzw. des Endoroboters relativ zum Körper bzw. relativ zum umgebenden Raum.

Auf diese Weise kann eine eindeutige Beziehung zwischen der Endoskopie-Kapsel bzw. des Endoroboters und der Anatomie hergestellt werden. Dies ist vor allem deshalb sinnvoll und wichtig, dass diagnostizierte Verletzungen bzw. pathologische Stellen des Darmes lokalisiert und auch von außen minimalinvasiv gefunden und therapiert werden können.

Die eindeutige Beziehung zwischen Position und Orientierung der Sensorspule 20 und der Anatomie wird durch ein konventionelles Registrierverfahren hergestellt. Dabei werden vom Anwender anatomische Landmarken am Patienten angewählt (Knochen, Organe), die in den die Messung unterstützenden anatomischen Aufnahmen unterschiedlicher Bildgebungsmodalitäten (C-Bogen, Ultraschall, Magnetresonanztomographie usw.) ebenso identifiziert werden können. Eine externe Navigations-Einheit 15 verknüpft rechnerisch das Signal der Sensorspule 20 mit dem durch die Registrierung definierten Koordinatensystem. Auf diese Weise ist es theoretisch möglich, die Position und Orientierung der Sensorspule 20 und damit der Endoskopie-Kapsel 1 bzw. des Endoroboters 2 mit einer Genauigkeit von ca. 1 mm im durch die Registrierung definiertem Koordinatensystem zu bestimmen. Praktisch ist allerdings nur eine orientierende Genauigkeit (je nach Patient und Untersuchungsbereich) von einigen Zentimetern gegeben, da wegen der stets gegebenen Organbewegung eine zeitabhängige Abweichung zwischen der Anatomie zum Zeitpunkt der Registrierung und zum Zeitpunkt der aktuellen Sensorspulenmessung besteht.

Das Signal der Sensorspule 19, welches die genaue Position und Orientierung im Wechselfeld definiert, kann entweder sofort an eine externe (das heißt außerhalb des Körpers befindliche) Endoskopie-Kapsel-Empfangseinheit bzw. Endoroboter-Empfangseinheit 13 gesendet werden, von dieser an die Navigations-Einheit 15 weitergeleitet und der Visualisierungs-Einheit 8 zugeführt werden, so dass zu jeder Endoskopie-Kapsel-Aufnahme bzw. Endoroboter-Aufnahme die dieser Aufnahme entsprechende Orientierung und Position der Endoskopie-Kapsel bzw. des Endoroboters möglicherweise auf dem gleichen Bildschirm gezeigt werden kann. Möglich wäre aber auch ein Abspeichern der Sensorspulensignale in einen Endoskopie-Kapsel- bzw. Endoroboter internen Speicherbaustein 19, welcher dann erst nach der genannten Untersuchung ausgewertet wird. In diesem Fall könnte auf eine Endoskopie-Kapsel- bzw. Endoroboter-Empfangseinheit verzichtet werden.

In einer weiteren Ausgestaltung der Erfindung wäre es denkbar, den Patienten den Magnetfeld-Generator sowie gegebenenfalls die Endoskopie-Kapsel bzw. die Endoroboter-Empfangseinheit über mehrere Stunden bzw. über Tage hinweg mit sich tragen zu lassen (beispielsweise an einem Gürtel). Sinnvoll ist dies, wenn dem Patienten der Zeitraum der Ruhelage nicht zugemutet werden kann, beispielsweise bei ausschließlicher Verwendung einer Endoskopie-Kapsel, deren Transport auf Basis der Peristaltik der Verdauungsorgane erfolgt und mehrere Stunden bzw. Tage umfassen kann.

Es sei angemerkt, dass die Frequenzen der bei Einsatz eines Endoroboters mit Magnetfeld-Steuerungssystem verwendeten 3D-Gradientenfelder im Bereich einiger weniger Hz liegen und das hochfrequente Wechselfeld (kHz) des Positionserkennungssystems in keiner Weise beeinflussen, geschweige denn beeinträchtigen dürften. Falls jedoch dennoch störende Interferenzen auftreten würden, wäre es möglich, die Niederfrequenz aus dem Hochfrequenz-Wechselfeld herauszufiltern.

## Patentansprüche

1. System zur In Vivo Positions- und Orientierungsbestimmung einer Endoskopie-Kapsel (1) im Rahmen einer kabellosen Endoskopie, aufweisend:
- einen Magnetfeld-Generator (12) zur Erzeugung eines elektromagnetischen HF-3D-Gradientenfeldes im zu untersuchenden Bereich,
- eine Endoskopie-Kapsel (1) mit integrierter Empfangs-Einheit (20) zur Ermittlung des jeweils aktuellen positionsund orientierungs-spezifischen HF-3D-Gradientenfeld-Wertes der Endoskopie-Kapsel (1),
- eine Navigations-Einheit (15) zum räumlichen Zuordnen des die Endoskopie-Kapsel-Position und -Orientierung definierenden aktuellen HF-3D-Gradientenfeld-Wertes, und
- eine Visualisierungs-Einheit (8) zur Darstellung der in einer definierten Endoskopie-Kapsel-Position und -Orientierung aufgenommenen anatomischen Bilder auf einem Bildschirm.

2. System nach Anspruch 1,
**dadurch gekennzeichnet,**
**dass** die Endokopie-Kapsel (1) als Endoroboter (2) ausgebildet ist.

3. System nach Anspruch 2 oder 3,
**dadurch gekennzeichnet,**
**dass** die Endoskopie-Kapsel (1) einen Speicherbaustein (19) aufweist welche die zu der jeweiligen anatomischen Bildaufnahme korrespondierenden positions- und orientierungsspezifischen HF-3D-Gradientenfeld-Werte abspeichert die dann von der Navigations-Einheit (15) gelesen werden.

4. System nach einem der Ansprüche 1 bis 3,
**dadurch gekennzeichnet,**
**dass** die Endoskopie-Kapsel (1) eine Sende-Einheit (4) aufweist mittels der die zu der jeweiligen anatomischen Bildaufnahme korrespondierenden positions- und orientierungsspezifischen HF-3D-Gradientenfeld-Werte an die Navigations-Einheit (15) gesendet werden.

5. System nach einem der vorhergehenden Ansprüche,
**dadurch gekennzeichnet,**
**dass** die integrierte Empfangs-Einheit (20) eine Sensor-Spule darstellt.

6. System nach einem der vorhergehenden Ansprüche,
**dadurch gekennzeichnet,**
**dass** der Magnetfeld-Generator (12) am Patienten (11) befestigt ist und von diesem über einen längeren Zeitraum mobil mitgeführt ist.

7. System nach einem der vorhergehenden Ansprüche,
**dadurch gekennzeichnet,**
**dass** das Navigationssystem (15) eine Zuordnung der anatomischen Bilder zur jeweiligen Position und Orientierung ermöglicht.

8. System nach einem der vorhergehenden Ansprüche,
**dadurch gekennzeichnet,**
**dass** die Visualisierungs-Einheit (8) die Darstellung der Endoskopie-Kapsel (1) erlaubt.

9. System nach einem der vorhergehenden Ansprüche,
**dadurch gekennzeichnet,**
**dass** die Visualisierungs-Einheit (8) eine parallele Darstellung von Endoskopie-Kapsel (1) und der bei dieser Position und Orientierung gewonnenen anatomischen Aufnahme ermöglicht.

10. Verfahren zur In Vivo Positions- und Orientierungsbestimmung einer Endoskopie-Kapsel (1) im Rahmen einer kabellosen Endoskopie
**gekennzeichnet durch** folgende Schritte:
- Erzeugen eines elektromagnetischen HF-3D-Gradientenfeldes im zu untersuchenden Bereich mittels einem Magnetfeld-Generator (12),
- Ermitteln des jeweils aktuellen positions- und orientierungs-spezifischen HF-3D-Gradientenfeld-Wertes der Endoskopie-Kapsel (1) mit einer integrierten Empfangs-Einheit (20),
- räumliches Zuordnen des aktuellen HF-3D-Gradientenfeld-Wertes mittels einer Navigations-Einheit (15), und
- Visualisieren der in einer definierten Position und Orientierung aufgenommenen anatomischen Bilder auf einem Bildschirm (8).

11. Verfahren nach Anspruch 10,
**dadurch gekennzeichnet,**
**dass** die Endoskopie-Kapsel (1) als Endoroboter (2) ausgebildet ist.

12. Verfahren nach Anspruch 10 oder 11,
**dadurch gekennzeichnet,**
**dass** die zu der jeweiligen anatomischen Bildaufnahme korrespondierenden positions- und orientierungs-spezifischen HF-3D-Gradientenfeld-Werte der Endoskopie-Kapsel (1) in einem Speicherbaustein (19) abgespeichert werden.

13. Verfahren nach einem der Ansprüche 10 bis 12,
**dadurch gekennzeichnet,**
**dass** die zu der jeweiligen anatomischen Bildaufnahme korrespondierenden positions- und orientierungs-spezifischen HF-3D-Gradientenfeld-Werte mittels einer in der Endoskopie-Kapsel (1) integrierten Sende-Einheit (4) an die Navigations-Einheit (15) gesendet werden.

14. Verfahren nach einem der Ansprüche 10 bis 13,
**dadurch gekennzeichnet,**
**dass** als integrierte Empfangs-Einheit (20) eine Sensor-Spule verwendet wird.

15. Verfahren nach einem der Ansprüche 10 bis 14,
**dadurch gekennzeichnet,**
**dass** auf der Visualisierungs-Einheit (8) auch die Darstellung der Endoskopie-Kapsel (1) erfolgt.

16. Verfahren nach einem der Ansprüche 10 bis 15,
**dadurch gekennzeichnet,**
**dass** der Magnetfeld-Generator (12) am Patienten (11) befestigt wird und von diesem über einen längeren Zeitraum mobil mitgeführt wird.

17. Verfahren nach einem der Ansprüche 10 bis 16,
**dadurch gekennzeichnet,**
**dass** mittels der Navigationseinheit (15) eine Zuordnung der anatomischen Bilder zur jeweiligen Endoskopie-Kapsel-Position und -Orientierung erfolgt.
